**Europäisches Patentamt**

**European Patent Office**

**Office européen des brevets**

(19)

(11) Publication number : **0 640 581 A1**

## (12) EUROPEAN PATENT APPLICATION

(21) Application number : **94306271.1**

(22) Date of filing : **25.08.94**

(51) Int. Cl.⁶ : **C07C 57/03, A61K 31/20**

(30) Priority : **25.08.93 JP 232298/93**

(43) Date of publication of application :
**01.03.95 Bulletin 95/09**

(84) Designated Contracting States :
**DE FR GB IT**

(71) Applicant : **BIOX CORPORATION**
**24-7, Higashinihonbashi 2-chome**
**Chuo-ku, Tokyo (JP)**

(71) Applicant : **SAGAMI CHEMICAL RESEARCH**
**CENTER**
**11-1, Marunouchi 1-chome**
**Chiyoda-ku**
**Tokyo 100 (JP)**

(72) Inventor : **Ueda, Shuichiro**
**2899-17, Kashiwabara**
**Sayama-shi, Saitama-ken (JP)**
Inventor : **Inada, Shin-ichi**
**Greenstage C102, 318-1**
**Tozaki. Ageo-shi, Saitama-ken (JP)**
Inventor : **Bukawa, Wakoto**
**332-20, Suna**
**Kawagoe-shi, Saitama-ken (JP)**
Inventor : **Kan, Tatsuhiko**
**239-41, Negishi**
**Sayama-shi. Saitama-ken (JP)**
Inventor : **Yazawa, Kazunaga**
**571, Unomori**
**Sagamihara-shi, Kanagawa-ken (JP)**

(74) Representative : **Marsden, John Christopher**
**Frank B. Dehn & Co.**
**Imperial House**
**15-19 Kingsway**
**London WC2B 6UZ (GB)**

(54) Endermic nutrient material and cosmetic composition containing the same.

(57)    An endermic nutrient material comprising as an effective component a saponified product of docosahexaenoic acid and/or a derivative thereof. A cosmetic composition containing such an endermic nutrient material is also disclosed. The cosmetic composition comprises i) at least one cosmetic component selected from fats and oils, waxes, hydrocarbons, higher fatty acids, alcohols, inorganic salts, dyes, pigments, perfumes, antiseptics and antioxidants, and ii) the saponified product of DHA and/or a derivative thereof, contained in an amount of 0.1% to 50% by weight.

The endermic nutrient material has the characteristic of permeating the skin and being absorbed therein, and being not liable to be oxidized. Hence, when cosmetics containing this endermic nutrient material are, for example, rubbed into the skin, it is possible for the DHA to be absorbed in human bodies by everyday and simple application, so that it can be expected to have an effect of various physiological activities possessed by the DHA and/or derivatives thereof and an effect of treating psoriasis and atopic dermatosis. A velvety and moist feel can also be imparted to the skin. Any fish odor undesirable for cosmetics can also be prevented.

EP 0 640 581 A1

## FIELD OF THE INVENTION

This invention relates to an endermic (percutaneously absorptive) nutrient material having as an effective component a saponified product of docosahexaenoic acid and/or a derivative thereof, and a cosmetic composition containing such an endermic nutrient material.

## BACKGROUND OF THE INVENTION

In recent years, various physiological activities possessed by docosahexaenoic acid (hereinafter "DHA") have attracted notice. For example, proposals are made on its application in a remedy for diabetic complication (Japanese Patent Application Laid-open No. 60-248610), a remedy for hemorrhoides (Japanese Patent Application Laid-open No. 61-24518), a lipoxynase metabolism stimulant (Japanese Patent Application Laid-open No. 63-230632), a carcinostatic (Japanese Patent Application Laid-open No. 1-153629), an antiallergic agent (Japanese Patent Application Laid-open No. 2-290812), a brain function improver (Japanese Patent Application Laid-open No. 1-290625), a remedy for cystic kidney (Japanese Patent Application Laid-open No. 2-235811) and a remedy for cardiac dysrhythmia (Japanese Patent Application Laid-open No. 4-29928).

As shown above, DHA is known to have various physiological activities, and various remedies utilizing such physiological activities of DHA are proposed. In all of these conventional remedies, DHA is orally or intraveneously administered so that the remedies can be efficacious.

However, some people dislike its oral administration because DHA produces an unpleasant odor similar to a fish odor especially when oxidized in the air. Meanwhile, its intraveneous administration must be conducted in medical facilities such as hospitals under supervision of doctors, and it has been difficult for DHA to have the intended efficacy by its everyday application.

## SUMMARY OF THE INVENTION

An object of the present invention is to provide an endermic (percutaneously absorptive) nutrient material that can make it possible to more effectively bring out the physiological activities possessed by DHA, and a cosmetic composition containing such an endermic nutrient material.

The present inventors have made researches for years on the use of DHA and/or derivatives thereof. In the course of their researches, they have discovered that a soap containing DHA and/or a derivative thereof have an effect of treating psoriasis, atopic dermatosis or the like and also an effect for making the skin velvety and moist, and have disclosed it in Japanese patent applications (see Japanese Patent Applications Laid-open No. 6-40887, No. 6-136393 and No. 6-136394, not laid open prior to the priority date of the present application).

The above Japanese applications, however, have no disclosure as to endermic properties possessed by saponified products of DHA and/or derivatives thereof and utilization of saponified products of DHA and/or derivatives thereof in cosmetics.

The present inventors made researches on the function possessed by DHA. As a result, they have discovered that saponified products of DHA and/or derivatives thereof have good endermic properties and that DHA and/or derivatives thereof having been saponified undergo oxidation with difficulty and become utilizable in cosmetics. Thus, on the basis of these facts, they have accomplished the present invention.

According to one aspect, the present invention provides an endermic nutrient material comprising as an effective component a saponified product of at least one of DHA and a derivative thereof.

According to another aspect, the present invention also provides a cosmetic composition comprising;

a cosmetic component selected from the group consisting of a fat or oil, a wax, a hydrocarbon, a higher fatty acid, an alcohol, an inorganic salt, a dye, a pigment, a perfume, an antiseptic and an antioxidant; and

a saponified product of at least one of DHA and a derivative thereof;

the saponified product of at least one of DHA and a derivative thereof being contained in an amount of from 0.1% by weight to 50% by weight based on the total weight of said cosmetic composition.

The present invention also provides a method of producing an endermic nutrient preparation, comprising mixing i) an endermic nutrient material comprising as an effective component a saponified product of at least one of docosahexaenoic acid and a derivative thereof with ii) a carrier, a diluent, an additive, or a combination thereof.

In the foregoing method, the endermic nutrient preparation may include the cosmetic composition described above, and the carrier, diluent, additive or combination thereof may be the cosmetic component described above.

The endermic nutrient material of the present invention comprises a saponified product of DHA and/or a derivative thereof as an effective component. This saponified product has the characteristic of permeating the skin and being absorbed therein as shown in Examples given later. Hence, its addition to preparations such as cosmetics used in contact with the skin makes it possible for the DHA and/or derivatives thereof to be absorbed into human bodies by everyday application, so that it can be expected to have an effect of various physiological activities possessed by the DHA and/or derivatives thereof and an effect of treating psoriasis and atopic dermatosis. The absorption of the DHA and/or derivatives thereof into the skin can also impart a velvety and moist feel to the skin.

The DHA itself has poor endermic properties, and no endermic properties can be exhibited unless DHA is converted into a saponified product. The saponified product of DHA, compared with the DHA itself, is not liable to be oxidized by oxygen in the air to bring about the advantages that it has a superior storage stability. Hence, any fish odor due to oxidation may occur with difficulty and it becomes possible to use it in cosmetics, which are often applied to faces.

## DESCRIPTION OF THE PREFERRED EMBODIMENTS

There are no particular limitations on the types of DHA or derivatives thereof used in the present invention, and any of a free acid DHA and its esters, glycerides, phospholipids, choline derivatives, amino acid derivatives and ascorbic acid derivatives can be used. As the DHA or its derivatives, not only those highly purified so as to contain only DHA but also those formed into partially purified oils containing other components such as eicosapentaenoic acid (EPA) may also be used. DHA can be produced by, for example, the process disclosed in Japanese Patent Application Laid-open No. 4-95048 by the present applicants.

The endermic nutrient material of the present invention can be produced by saponifying the DHA and/or a derivative thereof in the presence of an alkali agent preferably in an atmosphere of an inert gas such as nitrogen. The alkali agent used for the saponification may include any alkali agents conventionally used as saponifying agents, as exemplified by sodium hydroxide, potassium hydroxide, calcium hydroxide and triethanolamine.

The reason why the saponification is carried out in an atmosphere of an inert gas is that the DHA and/or derivatives thereof are prevented from being oxidized during the saponification as far as possible. The environment of an inert gas can be provided by, for example, a method in which the air present in the space in a reaction vessel or reaction apparatus is replaced with the inert gas. Such a method can be preferably employed.

The saponification may preferably be carried out at a temperature of from 50°C to 80°C, and more preferably from 50°C to 65°C. Saponification carried out at a temperature higher than 80°C tends to accelerate the oxidation of DHA and/or derivatives thereof. If on the other hand it is carried out at a temperature lower than 50°C, it becomes difficult to produce the saponified products. The alkali agent may preferably be added over a period of 1 hour or more as a time taken for its addition. Most preferably, the alkali agent may be added gradually over a full period of the time for saponification. The saponification mixture containing the saponified product thus obtained may be optionally neutralized with an organic acid such as citric acid or a mineral acid such as hydrochloric acid.

The endermic nutrient material according to one aspect of the present invention, comprises as an effective component the saponified product of DHA and/or a derivative thereof as described above. When used, this nutrient material may be mixed with a carrier, a diluent, an additive, or a combination thereof, e.g., mixed with water, in an appropriate concentration so that the resulting preparation can be applied to the skin. It may preferably be added to preparations such as cosmetics which are rubbed into the skin when used.

The cosmetic composition according to another aspect of the present invention is characterized by containing the DHA and/or a derivative thereof constituting the above endermic nutrient material. The cosmetic composition referred to in the present invention includes, for example, massage creams, cleansing creams, lotions, milky lotions, packs, hair tonics, and bath preparations.

In the cosmetic composition of the present invention, usual cosmetic components comprised of at least one component selected from a fat or oil, a wax, a hydrocarbon, a higher fatty acid, an alcohol, an inorganic salt, a dye, a pigment, a perfume, an antiseptic and an antioxidant are used as the component other than the saponified product of DHA and/or a derivative thereof. The saponified product of DHA and/or a derivative thereof must be contained in an amount of from 0.1% to 50% by weight, and preferably from 0.2% to 3% by weight, based on the total weight of the cosmetic composition.

In the above, the fat or oil used may include vegetable oils such as olive oil, almond oil and tsubaki oil, and animal oils such as beef tallow and fish oil. The wax used may include vegetable waxes such as carnauba wax, and animal waxes such as beeswax and lanolin. The hydrocarbon used may include paraffin, vaseline and squalene. The higher fatty acid used may include linolic acid. The alcohol used may include lower alcohols such as ethanol, isopropanol and butanol, and polyhydric alcohols such as glycerol, propylene glycol and ethylene glycol. The inorganic salt used may include sodium carbonate, sodium chloride and sodium sulfate. The pigment used may include zinc oxide, titanium oxide, talc, mica and kaolin. The antioxidant used may include tocopherol, BHA (butylated hydroxyanisole) and BHT (butylated hydroxytoluene). As the dye, the perfume and the antiseptic, those commonly used in cosmetics may be appropriately used.

EXAMPLES

Example 1

To 600 g of DHA mixed oil (DHA content: 23%; a product available from Tama Biochemical Co., Ltd.), 270 g of ethanol was added, which were then heated and mixed at 80°C in a reaction vessel while blowing nitrogen gas into it. To the resulting mixture, an aqueous solution separately prepared by dissolving 145 g of sodium hydroxide in 150 g of water was dropwise added little by little over a period of 30 to 60 minutes, and saponification was completed while keeping the temperature at 80°C. After the reaction was completed, while still keeping the temperature at 80°C, citric acid was added for neutralization to obtain a saponification product (a mixture).

Comparative Example 1

To the same DHA mixed oil as used in Example 1, a surface active agent and a thickening agent were added, followed by homogenization to obtain an emulsification product.

Experiment 1

With regard to the saponification product obtained in Example 1 and the emulsification product obtained in Comparative Example 1, their endermic properties were evaluated in the following way.

(1) Both arms of panelists were cleaned with commercially available toilet soap.

(2) The arms thus cleaned were further washed with hot water kept at 35 to 40°C, and gently wiped with a towel to remove the moisture.

(3) The saponification product obtained in Example 1 and the emulsification product obtained in Comparative Example were applied to the left arm and the right arm, respectively, in an amount of 5 ml for each so as to spread over the whole arms. In the saponification product and the emulsification product, the DHA mixed oil was mixed in the same amount based on the total weight.

(4) The both arms were each covered with a wrapping film, and kept warm at about 40°C for 1 hour.

(5) After the film was uncovered, the both arms were washed with hot water kept at 35 to 40°C to remove the saponification product and the emulsification product therefrom and then cleaned with commercially available toilet soap.

(6) The arms thus cleaned were further washed with hot water kept at 35 to 40°C, and gently wiped with a towel to remove the moisture.

(7) The both arms were washed with a disinfectant ethanol.

(8) The both arms thus washed was wiped with a gauze impregnated with ether, to collect fatty acids.

(9) The ether was evaporated from this gauze, and thereafter the fatty acids were analyzed by gas chromatography to measure the quantity of residual DHA on the outer skin.

As a control, the panelists' left arms to which neither the saponification product nor the emulsification product were applied was washed with a disinfectant ethanol and then wiped with a gauze impregnated with ether, to collect fatty acids. The ether was evaporated from this gauze, and thereafter the fatty acids were analyzed by gas chromatography to measure the quantity of residual DHA on the outer skin.

Results obtained are shown in Table 1.

Table 1

| | Quantity of residual DHA on the outer skin (based on fatty acid composition) |
|---|---|
| | (%) |
| Arms treated with the saponification product: | 29.84 |
| Arms treated with the emulsification product: | 9.91 |
| Control: | 0.1 |

As is seen from the results shown in Table 1, it has been ascertained that the saponification of the DHA mixed oil brings about an increase in the quantity of residual DHA on the outer skin.

Experiment 2

To 400 g of DHA mixed oil (DHA content: 27%; a product available from Tama Biochemical Co., Ltd.), 180 g of ethanol was added, which were then heated and mixed at 80°C in a reaction vessel kept in an atmosphere of nitrogen gas. To the resulting mixture, an aqueous solution separately prepared by dissolving 64 g of sodium hydroxide in 200 g of water was dropwise added little by little over a period of 30 to 60 minutes, and saponification was completed while keeping the temperature at 80°C. After the reaction was completed, while still keeping the temperature at 80°C, citric acid was added for neutralization to obtain a saponification product sample.

The sample thus obtained was put in a small-sized laboratory dish, which was then placed in a thermostat through which 70°C air was circulated, and its POV (peroxide value) was measured with time. For comparison, the POV of DHA mixed oil (DHA content: 27%) having not been saponified was also measured in the same way.

Results obtained are shown in Table 2.

The POV was measured according to a method described in "Standard Methods for the Analysis of Oils, Fats and Derivatives" (The Japan Oil Chemist's Society). In respect of the saponification product, the POV of fatty acids obtained by adding hydrochloric acid followed by extraction with ether and then dehydration was measured.

Table 2

| | | | | (meq/kg) |
|---|---|---|---|---|
| | After 0 day | After 1 day | After 4 days | After 7 days |
| DHA saponification product: | 1.8 | 2.52 | 5.1 | 10.2 |
| DHA mixed oil: | 1.05 | 10.2 | 73.0 | 75.1 |

As is seen from the results shown in Table 2, it has been ascertained that the saponification of DHA enables inhibition of the oxidation of DHA caused by oxygen in the air.

Example 2 (Cleansing cream)

Cleansing cream was prepared according to the formulation as shown in Table 3 below.

More specifically, borax was dissolved in pure water and thereafter the saponification product obtained in Example 1 was added. The resulting mixture was heated and kept at 70°C (an aqueous phase). Other components were mixed, and dissolved while heating. The solution obtained was kept at 70°C (an oily phase). The oily phase was added to the aqueous phase to carry out pre-emulsification, followed by uniform emulsification by means of a homomixer. After the emulsification was completed, the product was stirred while cooling. Thus, a cleansing cream containing the endermic nutrient material of the present invention was prepared.

5

Table 3

| | (by weight) |
|---|---|
| Solid paraffin | 4% |
| Microcrystalline wax | 8% |
| Beeswax | 6% |
| Vaseline | 0.8% |
| Liquid paraffin | 45% |
| Glycerol monooleate | 3.5% |
| Polyoxyethylene (20 mols) monooleate | 0.5% |
| Borax | 0.4% |
| Saponification product of Example 1 | 0.1% |
| Pure water | 24.2% |
| Perfume | 0.3% |
| Antiseptic | appropriate amount |

Example 3 (Lotion)

Lotion was prepared according to the formulation as shown in Table 4 below.

The saponification product obtained in Example 1, glycerol, polyethylene glycol and sodium hydroxide were dissolved in pure water at room temperature (an aqueous portion). Meanwhile, a perfume, an antiseptic and polyoxyethylene oleyl alcohol ether were dissolved in ethyl alcohol at room temperature (an alcohol portion). Then, the alcohol portion was added to the aqueous portion to carry out solubilization, and the resulting solution was colored with a dye, followed by filtration. Thus, a lotion containing the endermic nutrient material of the present invention was prepared. Final DHA concentration in this lotion was 0.3%.

Table 4

| | (by weight) |
|---|---|
| Saponification product of Example 1 | 3% |
| Glycerol | 10% |
| Polyyethylene glycol | 2% |
| Polyoxyethylene oleyl alcohol ether | 2% |
| Ethyl alcohol | 20% |
| Potassium hydroxide | 0.05% |
| Pure water | 62.75% |
| Perfume | 0.2% |
| Dye | appropriate amount |
| Antiseptic | appropriate amount |

Comparative Example 2

A lotion was prepared according to the same formulation and in the same manner as in Example 3 except that the saponification product obtained in Example 1 was replaced with a DHA mixed oil (DHA content: 23%) so added as to provide a final DHA concentration of 0.3%.

Experiment 3

The lotion of Example 3 and the lotion of Comparative Example 2 were each applied to seven panelists to have them evaluate a feel of moisture retention (a moist feel) after its use. Evaluation was made according to the criterions as shown in Table 5.
Results of the evaluation are shown in Table 6.

Table 5

| Marks | Evaluation |
|---|---|
| 1 | No moist feel at all. |
| 2 | A little moist feel. |
| 3 | A comparatively moist feel. |
| 4 | A reasonably moist feel. |
| 5 | A very moist feel. |

Table 6

| | Panelists | | | | | | | |
|---|---|---|---|---|---|---|---|---|
| | 1 | 2 | 3 | 4 | 5 | 6 | 7 | Average |
| **Final DHA concentration in lotion, 0.3%:** | | | | | | | | |
| Example 3 | 3 | 2 | 4 | 4 | 3 | 4 | 2 | 3.14 |
| (DHA saponified product) | | | | | | | | |
| Comparative Example 2 | | | | | | | | |
| (DHA mixed oil) | 2 | 1 | 2 | 2 | 1 | 2 | 1 | 1.57 |

As is seen from the above, the lotion of Example 3 that makes use of a DHA saponified product has a superior feel of moisture retension after its use, compared with the lotion of Comparative Example 2 that makes use of a DHA mixed oil

7

Example 4 (Cream)

Cream was prepared according to the formulation as shown in Table 7 below.

First, the saponification product of Example 1 and propylene glycol were added to pure water, and the mixture was heated so as to be kept at 70°C (an aqueous phase). Meanwhile, other components were mixed, and dissolved while heating. The solution obtained was kept at 70°C (an oily phase). The oily phase was added to the aqueous phase to carry out pre-emulsification, followed by uniform emulsification by means of a homomixer. After the emulsification was completed, the product was stirred while cooling. Thus, a cream containing the endermic nutrient material of the present invention was prepared.

Table 7

|  | (by weight) |
|---|---|
| Saponification product of Example 1 | 3% |
| Stearyl alcohol | 7% |
| Stearic acid | 2% |
| Hydrogenated lanolin | 2% |
| Squalene | 5% |
| 2-Octyldodecyl alcohol | 6% |
| Polyoxyyethylene cetyl alcohol ether | 3% |
| Glycerol monostearate | 2% |
| Propylene glycol | 5% |
| Pure water | 64.7% |
| Perfume | 0.3% |
| Antiseptic & antioxidant | appropriate amounts |

Example 5 (Milky lotion)

Milky lotion was prepared according to the formulation as shown in Table 8 below.

First, the saponification product of Example 1 and propylene glycol were added to pure water, and the mixture was heated and kept at 70°C (an aqueous phase). Meanwhile, other components were mixed, and dissolved while heating. The solution obtained was kept at 70°C (an oily phase). The oily phase was added to the aqueous phase to carry out pre-emulsification, followed by uniform emulsification by means of a homomixer. After the emulsification was completed, the product was stirred until it was cooled to 30°C. Thus, a milky lotion containing the endermic nutrient material of the present invention was prepared. Final DHA concentration in this lotion was 0.5%.

## Table 8

| | (by weight) |
|---|---|
| Saponification product of Example 1 | 5% |
| Microcrystalline wax | 1% |
| Beeswax | 2% |
| Lanoline | 2% |
| Liquid paraffin | 20% |
| Squalene | 10% |
| Sorbitan sesquioleate | 4% |
| Polyoxyethylene sorbitan monooleate | 1% |
| Propylene glycol | 7% |
| Pure water | 46% |
| Perfume | appropriate amount |
| Antiseptic | appropriate amount |

Comparative Example 3

A milky lotion was prepared according to the same formulation and in the same manner as in Example 5 except that the saponification product obtained in Example 1 was replaced with a DHA mixed oil (DHA content: 23%) so added as to provide a final DHA concentration of 0.5%.

Experiment 4

The milky lotion of Example 5 and the milky lotion of Comparative Example 3 were stored at a temperature of 70°C, and each applied to seven panelists to have them evaluate any fish odor after 0 day, after 1 day, after 4 days and after 7 days. Evaluation was made according to the criterions as shown in Table 9.

Results of the evaluation are shown in Table 10. In Table 10, numerical values indicate average values on the seven panelists.

Table 9

| Marks | Evaluation |
|---|---|
| 1 | No fish odor is conceivable at all. |
| 2 | A little fish odor is conceivable. |
| 3 | A fish odor is comparatively conceivable. |
| 4 | A fish odor is reasonably conceivable. |
| 5 | A fish odor is greatly conceivable. |

## Table 10

| | Days | | | |
|---|---|---|---|---|
| | 0 | 1 | 4 | 7 |
| Final DHA concentration in milky lotion, 0.5%: | | | | |
| Example 5 (DHA saponified product) | 1.00 | 1.00 | 1.20 | 1.20 |
| Comparative Example 3 (DHA mixed oil) | 1.00 | 1.30 | 2.00 | 2.50 |

As is seen from the above, the milky lotion of Example 5 that makes use of a DHA saponified product has prevented a fish odor from occurring, compared with the milky lotion of Comparative Example 2 that makes use of a DHA mixed oil.

Example 6 (Pack)

A pack was prepared according to the formulation as shown in Table 11 below.

Montmorillonite was dispersed in pure water and swelled. Thereafter, the saponification product of Example 1, liquid paraffin and propylene glycol were added and also zinc white and kaolin were also added, followed by further addition of ethyl alcohol in which a perfume and an antiseptic had been dissolved. The resulting mixture was stirred until it was formed into a uniform paste. Thus, a pack containing the endermic nutrient material of the present invention was prepared.

## Table 11

|  | (by weight) |
|---|---|
| Saponification product of Example 1 | 6% |
| Montmorillonite | 5% |
| Liquid paraffin | 2% |
| Propylene glycol | 5% |
| Zinc oxide | 10% |
| Kaolin | 10% |
| Ethyl alcohol | 5% |
| Pure water | 57% |
| Perfume | appropriate amount |
| Antiseptic | appropriate amount |

Example 7 (Hair tonic A)

A hair tonic was prepared according to the formulation as shown in Table 12 below.

Pure water, the saponification product of Example 1, and components other than the dye were added and dissolved in ethyl alcohol to carry out solubilization, and the resulting solution was colored with a dye, followed by filtration. Thus, a hair tonic containing the endermic nutrient material of the present invention was prepared.

Table 12

|  | (by weight) |
| --- | --- |
| Saponification product of Example 1 | 6% |
| Ethyl alcohol | 55% |
| Polyoxyethylene oleyl alcohol ether | 2% |
| Pure water | 37% |
| Perfume | appropriate amount |
| Salicylic acid | appropriate amount |
| Female sex hormone | appropriate amount |
| Hinokitiol | appropriate amount |
| Dye | appropriate amount |
| Ultraviolet absorbent | appropriate amount |

Example 8 (Hair tonic B)

A hair tonic was prepared according to the formulation as shown in Table 13 below.

Ethanol-soluble components (those from hinokitiol to a perfume solubilizing agent listed in Table 10) were successively added and dissolved in ethanol with stirring at room temperature. Meanwhile, the saponification product of Example 1 and propylene glycol were added and dissolved in pure water. Thereafter, the latter was added to the former ethanol phase so as to be uniformly mixed, followed by filtration. Thus, a hair tonic containing the endermic nutrient material of the present invention was prepared.

Table 13

|  | (by weight) |
| --- | --- |
| Hinokitiol | appropriate amount |
| Swertia extract | appropriate amount |
| Pyridoxine hydrochloride | appropriate amount |
| Acetic acid-$\alpha$-tocopherol | appropriate amount |
| Pantothenyl ethyl ether | appropriate amount |
| Perfume | appropriate amount |

| Perfume solubilizing agent | appropriate amount |
|---|---|
| Saponification product of Example 1 | 6% |
| Propylene glycol | 2% |
| Ethanol | 54% |
| Pure water | (total) 100% |

Example 9 (Massage cream)

Massage cream was prepared according to the formulation as shown in Table 14 below.

Formulation-A and Formulation-B were separately heat-melted at 70 to 80°C, followed by filtration. Using an emulsifying mixer, Formulation-A was mixed into Formulation-B with vigorous stirring. A perfume was added at around 45°C. Thus, a massage cream containing the endermic nutrient material of the present invention was prepared. The bleached beeswax and the spermaceti were added to adjust the consistency of the cream.

Table 14

| | (by weight) |
|---|---|
| Formulation-A: | |
| Saponification product of Example 1 | 3% |
| Liquid paraffin | 30% |
| Octyl dodecanol | 5% |

| | |
|---|---|
| Bleached beeswax | 4% |
| Spermaceti | 3% |
| Self-emulsifiable glycerol monostearate | 10% |
| Propyl parahydroxybenzoate | 0.1% |

Formulation-B:

| | |
|---|---|
| Sorbitol | 3% |
| Methyl parahydroxybenzoate | 0.15% |
| Pure water | 41.55% |
| Perfume | 0.2% |

Example 10 (Bath salts)

To 250 g of DHA mixed oil (DHA content: 23%; a product available from Tama Biochemical Co., Ltd.), 112 g of ethanol was added, which were then heated and mixed at 80°C in a reaction vessel kept in an atmosphere of nitrogen gas. To the resulting mixture, an aqueous solution separately prepared by dissolving 40 g of sodium hydroxide in 125 g of water was dropwise added little by little over a period of 30 to 60 minutes, and saponification was completed while keeping the temperature at 80°C. After the reaction was completed, while still keeping the temperature at 80°C, free alkali was adjusted, and the product was powdered to obtain a powdery saponification product.

While thoroughly mixing up 50 g of the above powdery saponification product, 400 g of sodium sesquicarbonate, 100 g of sodium chloride and 493 g of sodium sulfate using a blender, 5 g of liquid lanolin, 2 g of a dye and an appropriate amount of a perfume were added and uniformly mixed. Thus, bath salts containing the endermic nutrient material of the present invention was prepared.

Example 11 (Bath oil)

To 250 g of DHA mixed oil (DHA content: 23%; a product available from Tama Biochemical Co., Ltd.), 112 g of ethanol was added, which were then heated and mixed at 80°C in a reaction vessel kept in an atmosphere of nitrogen gas. To the resulting mixture, an aqueous solution separately prepared by dissolving 40 g of sodium hydroxide in 125 g of water was dropwise added little by little over a period of 30 to 60 minutes, and saponification was completed while keeping the temperature at 80°C. After the reaction was completed, while still keeping the temperature at 80°C, free alkali was adjusted to obtain a soft saponification product.

50 g of the above powdery saponification product, 300 g of hexadecyl alcohol, 230 g of polyethylene glycol, 70 g of polyoxyethylene (40 mols) hardened caster oil, and appropriate amounts of a perfume, a dye and an ultraviolet absorbent were mixed. Thus, a bath oil containing the endermic nutrient material of the present invention was prepared.

As described above, the saponified product of DHA and/or a derivative thereof contained in the endermic nutrient material has good endermic properties. Hence, the incorporation of the endermic nutrient material of the present invention into preparations such as cosmetics which are directly rubbed into the skin when used makes it possible for the DHA to be absorbed in human bodies by everyday and simple application, so that it can be expected to have an effect of various physiological activities possessed by the DHA and/or derivatives thereof and an effect of treating psoriasis and atopic dermatosis. When the cosmetics incorporated with the DHA and/or derivatives thereof are rubbed into the skin, a velvety and moist feel can be imparted to the skin. Also, the saponified product of DHA and/or a derivative thereof is not liable to be oxidized by oxygen and has a superior storage stability, and hence, any fish odor can be prevented from occurring when added to cosmetics.

**Claims**

1. An endermic nutrient material comprising one or more active ingredients selected from docosahexaenoic acid and derivatives thereof characterised in that said active ingredient is a saponification product.

2. An endermic nutrient material as claimed in claim 1 comprising one or more active ingredients selected from docosahexaenoic acid and esters, glycerides, phospholipid derivatives, choline derivatives, amino acid derivatives and ascorbic acid derivatives thereof.

3. A cosmetic composition comprising at least one cosmetic component selected from fats, oils, waxes, hydrocarbons, higher fatty acids, alcohols, inorganic salts, dyes, pigments, perfumes, antiseptics and antioxidants and an endermic nutrient material as claimed in claim 1 or claim 2, said endermic nutrient material being present in an amount of 0.1-50% by weight based on the total weight of said cosmetic composition.

4. A cosmetic composition as claimed in claim 3 in the form of a massage cream, cleansing cream, lotion, milky lotion, pack, hair tonic or bath preparation.

5. A process for the preparation of an endermic nutrient material as claimed in claim 1 which comprises saponifying a source of docosahexaenoic acid or a derivative thereof and optionally derivatising the product so obtained.

6. A process as claimed in claim 5 wherein the saponification is effected under an inert gas atmosphere.

7. A process as claimed in claim 5 or claim 6 wherein the endermic nutrient material is mixed with one or more carriers and/or diluents and/or additives to yield an endermic nutrient preparation.

8. A process as claimed in claim 7 wherein the endermic nutrient material is mixed with at least one cosmetic compound selected from fats, oils, waxes, hydrocarbons, higher fatty acids, alcohols, inorganic salts, dyes, pigments, perfumes, antiseptics and antioxidants to yield a cosmetic composition.

EP 0 640 581 A1

**European Patent Office**

# EUROPEAN SEARCH REPORT

Application Number

EP 94 30 6271

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,P, X | PATENT ABSTRACTS OF JAPAN vol. 18, no. 262 (C-1201) (6602) 19 May 1994 & JP-A-06 040 887 (SAGAMI CHEM.RES.CENTER) 15 February 1994 * abstract * | 1-4 | C07C57/03 A61K31/20 |
| X | EP-A-0 304 603 (INDENA S.P.A.) * page 4, line 53 - page 5, line 21 * * claims 1-10 * | 1-4 | |
| X | FR-A-2 648 347 (SEDERMA SA.) ENTIRE DOCUMENT. | 1-4 | |
| A | GB-A-2 181 349 (PARFUMS ROCHAS) ENTIRE DOCUMENT. | 1-4 | |
| | | | TECHNICAL FIELDS SEARCHED (Int.Cl.6) |
| | | | C07C A61K |

The present search report has been drawn up for all claims

| Place of search | Date of completion of the search | Examiner |
|---|---|---|
| THE HAGUE | 30 November 1994 | Klag, M |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

16